## Europäisches Patentamt
## European Patent Office
## Office européen des brevets

(19)

(11) Veröffentlichungsnummer: **0 013 561**
**A1**

(12) **EUROPÄISCHE PATENTANMELDUNG**

(21) Anmeldenummer: **80100035.7**

(22) Anmeldetag: **04.01.80**

(51) Int. Cl.³: **C 07 D 513/04**
C 07 B 19/00, A 61 K 31/425
//C07D233/42, (C07D513/04,
277/00, 235/00)

(30) Priorität: **05.01.79 CH 87/79**

(43) Veröffentlichungstag der Anmeldung:
**23.07.80 Patentblatt 80/15**

(84) Benannte Vertragsstaaten:
**AT BE CH DE FR GB IT LU NL SE**

(71) Anmelder: **CIBA-GEIGY AG**
**Patentabteilung Postfach**
**CH-4002 Basel(CH)**

(72) Erfinder: **Göschke, Richard, Dr.**
**Felixhäglistrasse 21**
**CH-4103 Bottmingen(CH)**

(72) Erfinder: **Ferrini, Pier Giorgio, Dr.**
**Im Rehwechsel 22**
**4102 Binningen(CH)**

(74) Vertreter: **Zumstein sen., Fritz, Dr. et al,**
**Bräuhausstrasse 4**
**D-8000 München 2(DE)**

(54) **Neue linksdrehende bicyclische Thia-diaza-Verbindung, Verfahren zu ihrer Herstellung, diese enthaltende pharmazeutische Präparate und ihre Verwendung.**

(57) Die Erfindung betrifft das linksdrehende Enantiomere des trans -5,6-Di-p -methoxyphenyl -2,3,5,6 -tetrahydro-imidazo [2,1-b] thiazols und seine pharmazeutische verwendbaren Salze, Verfahren zur Herstellung derselben, diese enthaltende pharmazeutische Präparate und ihre Verwendung.

Die neuen Verbindungen besitzen wertvolle pharmakologische Eigenschaften, insbesondere entzündungshemmende und antirheumatische Wirkungen.

EP 0 013 561 A1

0013561

- 1 -

4-11226/+/3/B

Neue linksdrehende bicyclische Thia-diaza-verbindung, Verfahren
zu ihrer Herstellung, diese enthaltende pharmazeutische Präparate
und ihre Verwendung

Die Erfindung betrifft das neue linksdrehende Enantiomere
des trans-5,6-Di-p-methoxyphenyl-2,3,5,6-tetrahydro-imidazo[2,1-b]-
thiazols und dessen Salze, sowie Verfahren zur Herstellung der genannten Verbindung und ihrer Salze, ferner pharmazeutische Präparate enthaltend die genannte Verbindung und ihre Salze sowie die Verwendung
der genannten Verbindung und ihrer Salze, vorzugsweise in Form von
pharmazeutischen Präparaten.

Salze des (-)-trans-5,6-Di-p-methoxy-2,3,5,6-tetrahydro-imidazo-
[2,1-b]thiazols sind insbesondere dessen Säureadditionssalze, insbesondere pharmazeutisch verwendbare, nicht-toxische Salze, z.B. mit
anorganischen Säuren, wie Chlorwasserstoff-, Bromwasserstoff-, Schwefel- oder Phosphorsäure, oder mit organischen Säuren, wie aliphatischen,
cycloaliphatischen, cycloaliphatisch-aliphatischen, aromatischen, araliphatischen, heterocyclischen oder heterocyclisch-aliphatischen
Carbon- oder Sulfonsäure, z.B. Essig-, Propion-, Bernstein-, Glykol-,
Milch-, Aepfel-, Wein-, Zitronen-, Ascorbin-, Malein-, Phenylessig-,
Benzoe-, 4-Aminobenzoe-, Anthranil-, 4-Hydroxybenzoe-, Salicyl-,
Aminosalicyl-, Embon- oder Nicotin-, sowie Methansulfon-, Aethansulfon-, 2-Hydroxyäthansulfon-, Aethylensulfon-, Benzolsulfon-, p-Toluolsulfon-, Naphthalinsulfon-, Sulfanil- oder Cyclohexylsulfaminsäure.

Die erfindungsgemässe Verbindung und ihre Salze besitzen wertvolle pharmakologische Eigenschaften, insbesondere entzündungshemmende
und antirheumatische Wirkungen, wie sich in Tierversuchen zeigen lässt.

- 2 -

z.B. im Kaolin-Pfotenoedem-Test (Helv. Physiol. Acta 25 (1967) 156) an
der Ratte bei einer peroral gegebenen Dosis ab etwa 10 mg/kg oder im
Terpentin-Pleuritis-Test [Helv. Physiol. Acta 26 (1969) 287] an der
Ratte peroral gegeben bei einer Dosis von 30 bis 100 mg/kg zeigen sie
eine antiinflammatorische bzw. anti-exsudative Wirkung. Sie zeigen
auch im Adjuvans-Arthritis-Test [Pharmacology 2 (1969) 288] an der
Ratte bei einer peroralen Dosis von 10-30 mg/kg eine ausgezeichnete
Wirkung.

Die neue Verbindung und ihre Salze sind auch analgetisch wirksam, wie sich im Phenyl-p-benzochinon-Test an der Maus (Proc. Soc.
Exp. Biol. 95 (1957) 729) bei Dosen von 30 bis 100 mg/kg, peroral gegeben, zeigen lässt.

Ferner ist die Hemmwirkung der neuen Verbindung und ihrer Salze
auf die Prostagladin-Synthetase in vitro [Prostaglandins 7, 123 (1974)]
in     Konzentrationen von 0,05-20 ug/ml zu nennen. Ausserdem zeigt
die neue Verbindung und ihre Salze einen vertvollen antithrombotischen
Effekt, nämlich einen Schutz vor tödlicher Lungenembolie an Kaninchen
[Pharmacology 14 (1976) 522] in peroralen Dosen von etwa 3 mg/kg.

Die neue Verbindung und ihre Salze zeigen zusätzlich dazu im
Pertussisoedem-Test (Agents and Actions, vol. 6, 613, 1976) bei etwa
3-10 mg/kg/Ratte eine verstärkende Wirkung.

Die neue Verbindung und ihre Salze können somit als Antiphlogistika z.B. zur Behandlung von rheumatischen, arthritischen und anderen mit Entzündungen verbundenen Erkrankungen, insbesondere von rheumatischer Arthritis, oder als Analgetika, z.B. zur Behandlung von
Schmerzzuständen, verwendet werden.

Die neue Verbindung kann nach an sich bekannten Methoden hergestellt werden.

- 3 -

So kann man z.B. das Enantiomerengemisch in die Enantiomeren aufspalten, das linksdrehende Enantiomere isolieren und, wenn erwünscht, die erhaltene freie Verbindung in ein Salz oder ein erhaltenes Salz in die freie Verbindung oder in ein anderes Salz umwandeln.

Die Aufspaltung des Enantiomerengemisches erfolgt in üblicher Weise, beispielsweise durch fraktionierte Kristallisation aus einem optisch aktiven Lösungsmittel, Chromatographie, insbesondere Dünnschichtchromatographie, an einem optisch aktiven Trägermaterial oder vorzugsweise durch Bildung von Salzen mit optisch aktiven Säuren, die mit trans-5,6-Di-p-methoxyphenyl-2,3,5,6-tetrahydro[2,1-b]thiazol Salze bilden, Auftrennung des diastereomeren Salzgemisches in die Diastereomeren auf Grund unterschiedlicher physikalischen Eigenschaften, wie der Löslichkeit oder des Adsorptionsverhaltens, z.B. durch fraktionierte Kristallisation oder Chromatographie, und Freisetzung des linksdrehenden Enantiomeren aus dem entsprechenden Diastereomeren in üblicher Weise, z.B. durch Basebehandlung, z.B. mittels Natriumhydrogencarbonat.

Mit trans-5,6-Di-p-methoxyphenyl-2,3,5,6-tetrahydro-imidazo-[2,1-b]thiazol diastereomere Salze bildende optisch aktive Säuren sind beispielsweise optisch aktive organische Säuren, vorzugsweise Mono- oder Dicarbonsäuren mit mindestens einem asymmetrischen C-Atom oder saure Ester bzw. saure Salze bzw. Amide von Dicarbonsäuren mit Alkoholen bzw. organischen Aminen, welche mindestens ein asymmetrisches C-Atom aufweisen, oder Sulfonsäuren mit mindestens einem asymmetrischen C-Atom. Geeignete Mono- und Dicarbonsäuren mit mindestens einem asymmetrischen C-Atom sind z.B. Camphersäure, Menthoxyessigsäure, Aepfelsäure, Mandelsäure und vor allem D- und L-Weinsäure und ihre O-Acylderivate sowie D- und L-Glutaminsäure und ihre N-Acyl- und N-Sulfonylderivate, O-Acylderivate der D- oder L-Weinsäure sind vorzugsweise O,O'-Diacylderivate derselben, beispielsweise solche, die sich von aliphatischen, cycloaliphatischen, cycloaliphatisch-aliphatischen, araliphatischen oder vor allem aromatischen Carbonsäuren
ableiten. Aliphatische Carbonsäuren sind beispielsweise Alkancarbonsäuren bzw. Alkan-dicarbonsäuren mit bis und mit 7 C-Atomen, z.B.
Essigsäure, Propionsäure, Acrylsäure oder Buttersäure. Cycloaliphatische
Carbonsäuren sind beispielsweise 5- bis 8-gliedrige Cycloalkancarbonsäuren, z.B. Cyclopentancarbonsäure, Cyclohexancarbonsäure oder Cycloheptancarbonsäure. Entsprechend sind cycloaliphatisch-aliphatische Carbonsäuren beispielsweise 5- bis 8-gliedrige Cycloalkylalkansäuren mit
bis und mit 4 C-Atomen in der Seitenkette, z.B. Cyclopentyl-,Cyclohexyl-
oder Cycloheptylessigsäure. Araliphatische Carbonsäuren sind beispielsweise im Phenylteil gegebenenfalls durch Alkyl mit bis und mit 4 C-
Atomen, wie Methyl, Alkoxy mit bis und mit 4 C-Atomen, wie Methoxy,
Halogen mit Atomnummer bis und mit 35, wie Chlor, und/oder Nitro substituierte Phenylalkansäuren mit bis und mit 4 C-Atomen in der Seitenkette, z.B. gegebenenfalls die wie angegeben substituierte Phenylessigsäure oder 2-Phenylpropionsäure. Aromatische Carbonsäuren sind beispielsweise durch Alkyl mit bis und mit 4 C-Atomen, wie Methyl, Alkoxy
mit bis und mit 4 C-Atomen, wie Methoxy, Halogen mit Atomnummer bis und
mit 35, wie Chlor, und/oder Nitro substituierte Benzoesäuren. Als 0,0'-
Diacylderivate der D- und L-Weinsäure kommen vorzugsweise deren 0,0'-
Di-p-toluolylderivate, ferner deren 0,0'-Dibenzoyl-, 0,0'-Di-o-toluoyl-,
0,0'-Di-m-toluoyl-, 0,0'-Di-p-Chlorbenzoyl-, 0,0'-Di-p-nitrobenzoyl-
oder 0,0'-Di-2,4-dinitrobenzoylderivate zur Anwendung. N-Acylderivate
der D- oder L-Glutaminsäure sind beispielsweise deren Lactame, z.B. D-
und L-Pyroglutaminsäure, oder N-Acylderivate, die sich vor aliphatischen, cycloaliphatischen, cycloaliphatisch-aliphatischen, araliphatischen oder aromatischen Carbonsäuren, vorzugweise von den vorstehend
genannten, ableiten. N-Sulfonylderivate der D- oder L-Glutaminsäure
sind vorzugweise solche, die sich von aromatischen Sulfonsäuren, ableiten, beispielsweise gegebenenfalls durch Alkyl mit bis und mit 4 C-
Atomen, wie Methyl, Alkoxy mit bis und mit 4 C-Atomen, wie Methoxy,
Halogen mit Atomnummer bis und mit 35, wie Fluor, Chlor oder Brom, und/
oder Nitro substituierte N-Benzolsulfonylderivate, namentlich N-Benzolsulfonyl-, N-p-Toluolsulfonyl-, p-Nitrobenzolsulfonyl-, p-Brombenzolsulfonyl-, oder p-Fluorbenzolsulfonylderivate der D- oder L-
Glutaminsäure.

Als optisch aktive Sulfonsäuren kommen z.B. Camphersulfonsäuren
in Betracht, wie Campher-10-sulfonsäure, 3-Brom-campher-3-sulfonsäure,
Campher-7-sulfonsäure oder 3-Brom-campher-10-sulfonsäure. Alkohole mit
mindestens einem asymmetrischen C-Atom sind beispielsweise aliphatische, araliphatische oder cycloaliphatische Alkohole, z.B. 1-Phenyl-
äthanol, Borneol, Isoborneol oder Terpineol. Organische Amine mit mindestens einem asymmetrischen C-Atom sind z.B. Cinchonin, Cinchonidin,
Chinin, Chinidin, Brucin, Ephedrin, Amphetamin and Menthylamin. Als
Dicarbonsäurekomponente für die Bildung saurer Ester und saurer Amide
bzw. Salze kommen z.B. Phthalsäure, Bernsteinsäure oder dergleichen
in Betracht.

Das als Ausgangsmaterial zu verwendende trans-5,6-Di-p-methoxy-
phenyl-2,3,5,6-tetrahydro[2,1-b]thiazol kann z.B. hergestellt werden,
indem man 2-Imino-3-(1,2-di-p-methoxyphenyl-2-hydroxy-äthyl)-thiazoli-
din der Säurebehandlung unterwirft, z.B. bei Raumtemperatur etwa 20
Stunden mit konzentrierter Schwefelsäure oder bei etwa 80°C etwa
3 Stunden mit Polyphosphorsäure behandelt,und das Rohprodukt erforderlichenfalls durch Umkristallisieren aus Toluol/Petroläther oder durch
Chromatographie an Silicagel mit Chloroform/Methanol (15:1) als Laufmittel reinigt. Das erwähnte 2-Imino-3-(1,2-di-p-methoxyphenyl-2-hydroxy-äthyl)-thiazolidin kann z.B. erhalten werden, indem man $\alpha$-Brom-
desoxyanisoin mit 2-Aminothiazolin kondensiert und in dem erhaltene
2-Imino-3-(1,2-di-p-methoxyphenyl-2-oxo-äthyl)-thiazolidin die Ketogruppe zu Hydroxymethylen reduziert, z.B. mittels Natriumborhydrid.

Die neue Verbindung kann man ferner herstellen, indem man
ein 2-(2-X-Aethylthio)-4,5-di-p-methoxyphenyl-4-imidazolin entsprechender, z.B. der (-)-threo-Konfiguration, worin X eine reaktionsfähige veresterte Hydroxygruppe darstellt, ringschliesst, und, wenn erwünscht, die erhaltenen freien Verbindungen in eines ihrer Salze überführt oder ein erhaltenes Salz in die freie Verbindung oder in ein anderes Salz umwandelt.

Reaktionsfähiges verestertes Hydroxy ist insbesondere mit einer starken anorganischen Säure, z.B. Halogenwasserstoff, insbesondere Chlorwasserstoff, oder Schwefelsäure, oder mit einer starken organischen Säure, wie mit einer Niederalkansulfonsäure, z.B. Methan- oder Aethansulfonsäure oder einer gegebenenfalls durch Niederalkyl, Niederalkoxy oder Halogen substituierten Benzolsulfonsäure, z.B. p-Toluolsulfonsäure oder p-Brombenzolsulfonsäure, verestertes Hydroxy.

Der Ringschluss wird vorzugsweise unter säureabspaltenden Bedingungen durchgeführt. Dabei arbeitet man in erster Linie in einem niedrigsiedenden Lösungsmittel, wie Dimethylformamid oder Aceton, einem Alkohol, z.B. Methanol oder Aethanol, wenn erwünscht, in Gegenwart einer Base, z.B. einer anorganischen Base, wie einem Alkali- oder Erdalkalihydrid, -hydroxyd oder -carbonat, in erster Linie Natriumhydrid, Natriumhydroxyd oder Natriumcarbonat, oder einer organischen Base, vorzugsweise einer Stickstoffbase, wie Triniederalkylamin, z.B. Trimethylamin, Triäthylamin, Dimethyl-isopropylamin, oder Pyridin.

Der Ausgangsstoff kann z.B. erhalten werden, indem man (-)-trans-2-Mercapto-4,5-di-p-methoxyphenyl-4-imidazolin und ein Aethylenglykol, worin mindestens eine der beiden Hydroxygruppen reaktionsfähig verestert ist, miteinander umsetzt und anschliessend, falls notwendig, die zweite Hydroxygruppe reaktionsfähig verestert. Die reaktionsfähig veresterten Hydroxygruppen entsprechen den obengenannten wie auch die Bedingungen der Kondensation. Bei dieser Reaktion können, insbesondere wenn beide Hydroxylgruppen reaktionsfähig verestert sind, der Ausgangsstoff in situ erhalten werden, welcher ohne Isolierung zum Ring geschlossen werden kann.

Das als Ausgangsstoff zu verwendende trans-2-Mercapto-4,5-di-
p-methoxyphenyl-imidazolin kann z.B. erhalten werden, indem man p-
Anisoindioxim durch Umsetzung mit einem unedlen Metall, wie einem
Alkalimetall und einem Alkohol, z.B. mit Natrium und einem Niederalkanol, wie Aethanol oder Methanol, oder einem Erdalkalimetall,
Zinz oder Eisen und einer Protonensäure, z.B. mit Zink und Essigsäure oder Eisen und Salzsäure, zu D,L-1,2-Di-p-methoxyphenyl-äthylendiamin reduziert,dieses in die Antipoden aufspaltet und das Enantiomere
der gewünschten Konfiguration mit Schwefelkohlenstoff umsetzt.

**Die neue Verbindung kann ferner erhalten werden, indem man ein
2-Imino-3-(2-X-1,2-di-p-methoxyphenyl-äthyl)-thiazolidin geeigneter
Konfiguration, worin X gegebenenfalls reaktionsfähiges verestertes
Hydroxy bedeutet, oder ein Tautomeres davon ringschliesst, und wenn er-**
wünscht, die erhaltene freie Verbindung in eines ihrer Salze überführt
oder ein erhaltenes Salz in die freie Verbindung umwandelt.

Reaktionsfähig verestertes Hydroxy ist insbesondere eine mit
einer starken anorganischen Säure, z.B. Halogenwasserstoff, insbeson--
dere Chlorwasserstoff, oder Schwefelsäure, oder mit einer starken
organischen Säure, wie mit einer Niederalkansulfonsäure, z.B. Methan-
oder Aethansulfonsäure oder einer gegebenenfalls durch Niederalkyl,
Niederalkoxy oder Halogen substituierten Benzolsulfonsäure, z.B. p-
Toluolsulfonsäure oder p-Brombenzolsulfonsäure veresterte Hydroxygruppe. X ist jedoch in erster Linie eine freie Hydroxygruppe.

Der Ringschluss erfolgt in an sich bekannter Weise, erforderlichenfalls in Gegenwart eines Kondensationsmittels und/oder unter
Erwärmen, z.B. auf etwa 50 bis 150°C, vorzugsweise in einem inerten
Lösungsmittel. Als Kondensationsmittel kommen ausgehend von Ausgangsstoffen, worin X Hydroxy ist, z.B. saure und ausgehend von Ausgangsstoffen, worin X reaktionsfähiges verestertes Hydroxy ist, z.B. basische Kondensationsmittel in Betracht. Saure Kondensationsmittel sind
z.B. starke Lewis-Säuren, z.B. Schwefelsäure oder Polyphosphorsäure.
Basische Kondensationsmittel sind z.B. anorganische oder organische

- 8 -

Basen, wie Natrium- oder Kaliumhydroxyd, Pyridin oder Di-Isopropyläthylamin. Inerte Lösungsmittel sind beispielsweise Acetonitril oder
Alkohole, z.B. Methanol oder Aethanol. Dementsprechend erfolgt der
Ringschluss, falls X eine freie Hydroxygruppe ist, vorzugsweise unter
wasserabspaltenden Bedingungen, z.B. bei etwa 50° bis etwa 150°, und/
oder in Gegenwart eines sauren Katalysators, wie Schwefelsäure oder
Polyphosphorsäure. Die Reaktion kann auch in Gegenwart eines Lösungsmittels erfolgen. Ist X eine reaktionsfähig veresterte Hydroxygruppe
erfolgt der Ringschluss z.B. in Gegenwart von Natrium- oder Kaliumhydroxyd, Pyridin oder Di-Isopropyläthylamin unter wasserabspaltenden
Bedingungen, wie durch Erwärmen, z.B. von etwa 50° bis etwa 150°, vorzugsweise in Gegenwart eines Lösungsmittels, wie Acetonitril oder einem
Alkohol, z.B. Methanol oder Aethanol.

Der Ringschluss erfolgt, falls X eine freie Hydroxygruppe ist,
vorzugsweise unter wasserabspaltenden Bedingungen, z.B. bei etwa
50° bis etwa 150° und/oder in Gegenwart eines sauren Katalysators,
wie starke Lewis-Säuren, z.B. Schwefelsäure oder Polyphosphorsäure.
Die Reaktion kann auch in Gegenwart eines Lösungsmittels erfolgen.
Ist X eine reaktionsfähig veresterte Hydroxygruppe erfolgt der Ringschluss z.B. in Gegenwart einer anorganischen oder organischen Base,
wie Natrium- oder Kaliumhydroxyd, Pyridin oder Di-Isopropyläthylamin.

Die Ausgangsstoffe lassen sich z.B. erhalten, indem man in an
sich bekannter Weise ein 2-Oxo-1,2-di-p-methoxyphenyl-1-halogen-äthan
geeigneter Konfiguration mit 2-Amino-thiazolin oder dessen Tautomeren
umsetzt, das erhaltene kristalline Produkt isoliert, die Ketogruppe
mit einem Dimetallhydrid zur Hydroxygruppe reduziert und ein gegebenenfalls erhaltenes Diastereomerengemisch in üblicher Weise, z.B. durch
fraktionierte Kristallisation, in die Komponenten auftrennt. Die Hydroxygruppe kann dann gewünschtenfalls in üblicher Weise in einen reaktionsfähigen Ester übergeführt werden.

- 9 -

Die obengenannte Kondensation erfolgt vorzugsweise bei niederer Temperatur, wie -20° bis +30°, insbesondere bei Raumtemperatur, vorzugsweise in einem Lösungsmittel, wie Acetonitril oder einem aromatischen Kohlenwasserstoff, wie Benzol oder Toluol,oder einem halogenierten aliphatischen Kohlenwasserstoff, z.B. Chloroform oder Methylenchlorid. Das Kondensationsprodukt fällt nach kurzer Zeit,meist nach etwa 15 bis 45 Minuten, aus und wird dann sofort abgenutscht.

Die Reduktion der Ketogruppe wird vorzugsweise in Lösung oder Suspension in einem niederen Alkohol, wie Methanol oder Aethanol bei niederer Temperatur, d.h. ungefähr -20° bis +20°,durchgeführt. Als Dimetallhydride verwendet man solche die eine Ketogruppe reduzieren können, vorzugsweise Natriumborhydrid. Die Reduktionszeit kann sehr lange dauern je nach der Kristallgrösse in der Suspension. Mit einer Zeit von 2- bis ungefähr 42 Stunden muss gerechnet werden.

Infolge der engen Beziehungen zwischen der neuen Verbindung in freier Form und in Form ihrer Salze sind im Vorausgegangenen und nachfolgend unter der freien Verbindung oder den Salzen sinn- und zweckmässig gegebenenfalls auch die entsprechenden Salze bzw. freie Verbindung zu verstehen.

Die obigen Reaktionen werden in üblicher Weise in An- oder Abwesenheit von Verdünnungs-, Kondensations- und/oder katalytischen Mitteln, falls notwendig, bei erniedrigter oder erhöhter Temperatur, im geschlossenen Gefäss und/oder in einer Inertgasatmosphäre durchgeführt.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder

- 10 -

während der Reaktion gebildet werden.

Die Erfindung betrifft auch die als Zwischenprodukte erwähnten diastereomeren Salze des linksdrehenden Enantiomeren von trans-5,6-Di-p-methoxyphenyl-2,3,5,6-tetrahydro-imidazo[2,1-b]thiazol mit optisch aktiven Säuren, vorzugsweise mit organischen Mono- oder Dicarbonsäuren, die mindestens ein asymmetrisches C-Atom aufweisen, beispielsweise mit D- oder L-Weinsäure bzw. deren 0,0'-Diacylderivaten, namentlich Salze mit (+)-Di-0.0'-p-toluoyl-D-weinsäure bzw. (-)-Di-0,0'-p-toluoyl-L-weinsäure.

In den eingangs definierten biologischen Testanordnungen sind sie ungefähr gleich wirksam wie die freie Base bzw. deren eingangs erwähnten Säureadditionssalze und können als Antiinflammatorika, z.B. zur Behandlung von rheumatischer Arthritis, verwendet werden.

Die neuen Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zur enteralen oder parenteralen Verabreichung eignen. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/ oder Glycerin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-,Weizen-, Reis- oder Pfeilwurzstärke, Gelatine,Tragakanth,Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmacksstoffe und Süssmittel. Ferner kann man die neuen pharmakologisch wirksamen Verbindungen in Form von injizierbaren, z.B. intravenös verabreichbaren Präparaten oder von Infusionslösungen verwenden. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden lönnen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffes. Die Einzeldosis für einen Warmblüter von etwa 70 kg Gewicht beträgt zwischen 0,1 und 0,75 g, die Tagesdosis zwischen 0,2 und 1,0 g.

Die folgenden Beispiele dienen zur Illustration der Erfindung; Temperaturen werden in Celsiusgraden angegeben.

Beispiel 1: Eine Suspension von 7 ml 1,2-Dibromäthan, 7 g Natriumcarbonat und 55 ml Isopropanol wird bei Raumtemperatur gerührt und innert einer Stunde mit der Suspension von 4,7 g trans-4,5-Di-p-methoxyphenyl-imidazolidin-2-thion in 110 ml 1,5%-iger Natronlauge versetzt. Das Reaktionsgemisch wird 7 Stunden am Rückfluss gekocht, dann werden das Isopropanol und das Dibromäthan am Rotationsverdampfer entfernt und die verbleibende Suspension wird mit Toluol extrahiert. Der Toluolextrakt wird mit Sole gewaschen, über Natriumsulfat getrocknet und eingedampft. Der Rückstand wird auf Silicagel chromatographiert. Nach dem Abtrennen unpolarer Verunreinigungen mit Aethylacetat wird das

- 12 -

trans-5,6-Di-p-Methoxyphenyl-2,3,5,6-tetrahydro-imidazo[2,1-b]thiazol
mit einem Gemisch aus Aethylacetat/Methanol (99:1) als farbloses Oel
eluiert. Es kristallisiert spontan zu weissen Kristallen, vom Smp.
125-126°.

Zur weiteren Reinigung wird das Material zwischen Essigester
und 2-n Salzsäure verteilt. Die sauren Phasen werden mit 1n-Natrium-
hydrogencarbonat auf pH 8 gestellt und mit Essigester extrahiert.
Nach dem Trocknen und Eindampfen kristallisiert man den Rückstand
aus Toluol/Petrol/Aether um oder chromatographiert an Silicagel mit
Chloroform/Methanol (15:1) als Laufmittel.

Das Ausgangsmaterial kann beispielsweise folgendermassen hergestellt werden: · ··

67,7 g D,L-1,2-Di-p-methoxyphenyl-äthylendiamin, erhältlich
durch Reduktion von p-Anisoindioxim mit Natrium in Aethanol, werden in
1400 ml Aethanol gelöst, mit 27 g Schwefelkohlenstoff versetzt und
16 Stunden zum Rückfluss erhitzt. Die ausgefallenen Kristalle werden
abgenutscht, in 1200 ml Aethanol aufgeschlämmt und zum Rückfluss erhitzt, bis sich kein Schwefelwasserstoff mehr entwickelt (etwa 30
Stunden). Man erhält das trans-4,5-Di-p-methoxyphenyl-imidazolidin-2-
thion, das ohne weitere Reinigung verwendet werden kann.

Beispiel 2:  6,8 g trans-5,6-Di-p—methoxyphenyl-2,3,5,6-tetrahydro-
imidazo[2,1-b]thiazol werden in 80 ml Chloroform gelöst und eine
Lösung von 8,1 g (+)-Di-0,0'-p-toluoyl-D-weinsäure in 80 ml Chloroform
zugegeben. Man dampft am Rotationsverdampfer bei 50° zur Trockne ein
und nimmt den Rückstand in 50 ml warmem Aceton auf, rührt bei Raumtemperatur und lässt 4 Stunden stehen. Die ausgefallenen Kristalle werden
abfiltriert, mit Aceton nachgewaschen und bei 70° im Vakuum getrocknet.
Das so erhaltene (+)-Di-p-toluoyl-D-tartrat des (-)-Enantiomeren von
trans-5,6-Di-p-methoxyphenyl-2,3,5,6-tetrahydro-imidazo[2,1-b]thiazol schmilzt nach nochmaligem Umkristallisieren bei 126-128°, $[\alpha]_D^{20°} = -5$.

Beispiel 3: 11,0 g trans-5,6-Di-p-methoxy-phenyl-2,3,5,6-tetrahydro-imidazo[2,1-b]thiazol werden in 120 ml Chloroform gelöst und eine Lösung von 13,1 g (-)-Di-0,0'-p-toluoyl-L-weinsäure in 120 ml Chloroform zugegeben. Man dampft am Rotationsverdampfer bei 50° zur Trockne ein, nimmt den öligen Rückstand in 80 ml Aceton unter Erwärmen auf, und lässt bei Raumtemperatur 4 Stunden stehen. Die ausgefallenen Kristalle werden abgenutscht und mit Aceton nachgewaschen.

Das Filtrat und die Waschlösung werden zur Trockne gedampft, der Rückstand in 35 ml Aceton gelöst und über Nacht im Eisschrank stehen gelassen. Die erhaltenen Kristalle, das Di-p-toluoyl-L-tartrat des (-)-Enantiomeren von trans-5,6-Di-p-methoxyphenyl-2,3,5,6-tetrahydro-imidazo[2,1-b]thiazol schmelzen nach Umkristallisieren aus Aceton bei 118-120° $[\alpha]_D^{20°} = -160°$.

Beispiel 4: 17,0 g trans-5,6-Di-p-methoxyphenyl-2,3,5,6-tetrahydro-imidazo[2,1-b]thiazol werden in 100 ml warmem Aceton gelöst. Ebenso werden 7,5 g L-(+)-Weinsäure in 150 ml warmem Aceton gelöst. Nach Abkühlen der Lösungen auf Ramtemperatur werden diese vereinigt, wobei sich ein klebriger Niederschlag bildet. Dann wird unter Rühren langsam bis zur Auflösung desselben gerührt. Dann lässt man unter gelegentlichem Schütteln langsam abkühlen, bis Kristallisation erfolgt. Man lässt 3,5 Stunden bei Raumtemperatur stehen, dekantiert von den ausgeschiedenen Kristallen ab und wäscht diese mit Aceton nach.

Die abdekantierte Mutterlauge wird zur Trockne eingedampft und der Rückstand mit n-Natriumhydrogencarbonatlösung auf pH=8 gestellt. Dann wird dreimal mit Diäthyläther ausgezogen. Die Auszüge werden vereinigt, zweimal mit Wasser und einmal mit gesättigter Kochsalzlösung gewaschen, über Natriumsulfat getrocknet und unter vermindertem Druck eingedampft.

Der Eindampfrückstand wird in 100 ml Aceton gelöst und bei Raumtemperatur mit einer Lösung von 3,7 g D-(-)-Weinsäure in 150 ml Aceton versetzt. Das Gemisch wird gelinde erwärmt, bis eine klare Lösung entsteht. Dann lässt man langsam Abkühlen, wobei Kristallisation erfolgt. Man lässt 3 Stunden bei Raumtemperatur stehen, saugt die ausgefallenen Kristalle ab, wäscht mit Aceton und trocknet unter vermindertem Druck bei 60°. Man erhält das D-(-)-Tartrat des (-)-Enantiomeren von trans-5,6-Di-p-methoxyphenyl-2,3,5,6-tetrahydro-imidazo[2,1-b]-thiazol vom Smp. 125-127°, $[\alpha]_D^{20}$ = -115°.

Beispiel 5: 6,5 g des D-(-)-Tartrats des (-)-Enantiomeren von trans-5,6-Di-p-methoxy-phenyl-2,3,5,6-tetrahydro-imidazo[2,1-b]thiazol werden in 25 ml Wasser von Raumtemperatur gelöst und mit 50 ml 1-n wässeriger Natriumhydrogencarbonatlösung versetzt. Man extrahiert zweimal mit Aether, wäscht die Aetherextrakte mit Wasser, trocknet sie über Natriumsulfat, filtriert und dampft ein. Man erhält so das (-)-Enantiomere des trans-5,6-Di-p-methoxy-phenyl-2,3,5,6-tetrahydro-imidazo-[2,1-b]thiazol vom $[\alpha]_D^{20°}$ = -44°.

Beispiel 6: 2,0 g des (-)-Enantiomeren des trans-5,6-Di-p-methoxy-phenyl-2,3,5,6-tetrahydro-imidazo[2,1-b]thiazols werden in 20 ml Aceton gelöst und eine Lösung von 0,9 g D-(-)-Weinsäure in 50 ml Aceton zugegeben. Man engt auf ca. 30 ml ein. Die langsam ausfallenden Kristalle werden abgenutscht, mit Aceton nachgewaschen und bei 80° am Hochvakuum getrocknet. Man erhält so das D-(-)-Tartrat des (-)-Enantiomeren von trans-5,6-Di-p-methoxy-phenyl-2,3,5,6-tetrahydro-imidazo[2,1-b]-thiazols, vom F. = 122-125° $[\alpha]_D^{20}$ = -120°.

Beispiel 7: Tabletten, enthaltend 25 mg Wirkstoff, z.B. (-)-trans—5,6-Di-p-methoxyphenyl-2,3,5,6-tetrahydro-imidazo[2,1-b]thiazol können folgendermassen hergestellt werden:

- 15 -

Bestandteile (für 1000 Tabletten)

| | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung:

Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben. Der erhaltenen Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 25 mg einer anderen der in den Beispielen 1 bis 6 genannten Verbindung hergestellt werden.

Beispiel 8: Kautabletten, enthaltend 30 mg Wirkstoff, z.B. (-)-trans-5,6-Di-p-methoxyphenyl-2,3,5,6-tetrahydro-imidazo[2,1-b]thiazol, können z.B. folgendermassen hergestellt werden:

Zusammensetzung (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |

| | |
|---|---|
| Stearinsäure | 10,0 g |
| Saccharin | 1,0 g |
| 5%-ige Gelatinelösung | q.s. |

Herstellung:

Alle festen Ingredienzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst.

In analoger Weise können auch Kautabletten, enthaltend jeweils 30 mg einer anderen der in den Beispielen 1 bis 6 genannten Verbindungen hergestellt werden.

Beispiel 9: Tabletten enthaltend 100 mg Wirkstoff, z.B. (-)-trans-5,6-Di-p-methoxyphenyl-2,3,5,6-tetrahydro-imidazo[2,1-b]thiazol können folgendermassen hergestellt werden:

Zusammensetzung (für 1000 Tabletten):

| | |
|---|---|
| Wirkstoff | 100,0 g |
| Lactose | 248,5 g |
| Maisstärke | 17,5 g |
| Polyäthylenglykol 6000 | 5,0 g |
| Talkum | 15,0 g |
| Magnesiumstearat | 4,0 g |
| entmineralisiertes Wasser | q.s. |

Herstellung:

Die festen Ingedienzien werden zunächst durch ein Sieb mit
0,6 mm Maschenweite getrieben. Dann werden Wirkstoffe, Lactose, Talkum,
Magnesiumstearat und die Hälfte der Stärke innig vermischt. Die andere
Hälfte der Stärke wird in 65 ml Wasser suspendiert und diese Suspension
zu einer siedenden Lösung des Polyäthylenglykols in 260 ml Wasser
hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei
35° getrocknet, durch ein Sieb mit 1,2 mm Maschenweite getrieben und
zu beidseitig konkaven Tabletten von etwa 10 mm Durchmesser mit Bruchkerbe auf der Oberseite verpresst.

In analoger Weise können auch Tabletten, enthaltend 100 mg
einer anderen Verbindung gemäss einem der Beispiele 1-6 hergestellt
werden.

Patentansprüche
(für alle benannten Länder ausser Oesterreich)

1.      Das linksdrehende Enantiomere des trans-5,6-Di-p-methoxy-phenyl-2,3,5,6-tetrahydro-imidazo[2,1-b]thiazols oder ein pharmazeutisch verwendbares Säureadditionssalz mit einer anorganischen oder organischen Säure.

2.      Das linksdrehende Enantiomere des trans-5,6-Di-p-methoxy-phenyl-2,3,5,6-tetrahydro-imidazo[2,1-b]thiazols in freier Form.

3.      Das linksdrehende Enantiomere des trans-5,6-Di-p-methoxyphenyl-2,3,5,6-tetrahydro-imidazo[2,1-b]thiazols in Form des Chlorwasser-stoffsäure-, Bromwasserstoffsäure-, Schwefelsäure- oder Phosphorsäure-salzes oder eines Salzes mit D- oder L-Weinsäure bzw. eines von der gegebenenfalls durch $C_1$-$C_4$-Alkyl, $C_1$-$C_4$-Alkoxy, Halogen bis und mit Atomnummer 35 und/oder Nitro substituierten Benzoesäure abgeleiteten 0,0'-Diacylderivates derselben.

4.      Das linksdrehende Enantiomere des trans-5,6-Di-p-methoxyphenyl-2,3,5,6-tetrahydro-imidazo[2,1-b]thiazols in Form des D-Tartrates.

5.      Das linksdrehende Enantiomere des trans-5,6-Di-p-methoxyphenyl-2,3,5,6-tetrahydro-imidazo[2,1-b]thiazols in Form des Di-p-Toluoyl-D-tartrates.

6.      Das linksdrehende Enantiomere des trans-5,6-Di-p-methoxyphenyl-2,3,5,6-tetrahydro-imidazo[2,1-b]thiazols in Form des Di-p-Toluol-L-tartrates.

7.      Pharmazeutische Präparate enthaltend eine Verbindung gemäss einem der Ansprüche 1 bis 6 neben üblichen pharmazeutischen Hilfs- und Trägerstoffen.

8.    Verfahren zur Herstellung des linksdrehenden Enantiomeren des trans-5,6-Di-p-methoxyphenyl-2,3,5,6-tetrahydro-imidazo[2,1-b]thiazols und seiner pharmazeutisch verwendbaren Säureadditionssalze, dadurch gekennzeichnet, dass man das Enantiomerengemisch in die Enantiomeren aufspaltet und das linksdrehende Enantiomere isoliert oder ein 2-(2-X-Aethylthio)-4,5-di-p-methoxyphenyl-4-imidazolin entsprechender Konfiguration, worin X eine reaktionsfähige veresterte Hydroxygruppe darstellt, oder ein 2-Imino-3-(2-X-1,2-di-p-methoxyphenyl-äthyl)-thiazolidin geeigneter Konfiguration oder ein Tautomeres davon ringschliesst und, wenn erwünscht, die gegebenenfalls in freier Form erhaltene Verbindung in eines ihrer Salze oder ein erhaltenes Salz in die freie Verbindung oder in ein anderes Salz überführt.

9.    Verfahren nach Anspruch 8, dadurch gekennzeichnet, dass man die Aufspaltung des Enantiomerengemisches durch Bildung eines diastereomeren Salzes mit einer optisch aktiven Säure, die mit trans-5,6-Di-p-methoxyphenyl-2,3,5,6-tetrahydro-imidazo[2,1-b]thiazol Salze bilden kann, Auftrennung des diastereomeren Salzgemisches in die Diastereomeren und Freisetzung des gewünschten Enantiomeren bewirkt.

10.    Eine Verbindung gemäss einem der Ansprüche 1- 7 zur Verwendung bei der prophylaktischen und/oder therapeutischen Behandlung rheumatischer, arthritischer oder anderer mit Entzündungen verbundenen Erkrankungen.

Patentansprüche

(für Oesterreich)

1.      Verfahren zur Herstellung des linksdrehenden Enantiomeren des
trans-5,6-Di-p-methoxyphenyl-2,3,5,6-tetrahydro-imidazo[2,1-b]thiazols
und seiner pharmazeutisch verwendbaren Säureadditionssalze, dadurch
gekennzeichnet, dass man das Enantiomerengemisch in die Enantiomeren
aufspaltet und das linksdrehende Enantiomere isoliert oder ein 2-(2-X-
Aethylthio)-4,5-di-p-methoxyphenyl-4-imidazolin entsprechender Konfiguration, worin X eine reaktionsfähige veresterte Hydroxygruppe darstellt, oder ein 2-Imino-3-(2-X-1,2-di-p-methoxyphenyl-äthyl)-thiazoli-
din geeigneter Konfiguration oder ein Tautomeres davon ringschliesst
und, wenn erwünscht, die gegebenenfalls in freier Form erhaltene Verbindung in eines ihrer Salze oder ein erhaltenes Salz in die freie
Verbindung oder in ein anderes Salz überführt.

2.      Verfahren nach Anspruch 1, dadurch gekennzeichnet, dass man
die Aufspaltung des Enantiomerengemisches durch Bildung eines diastereomeren Salzes mit einer optisch aktiven Säure, die mit trans-
5,6-Di-p-methoxyphenyl-2,3,5,6-tetrahydro-imidazo[2,1-b]thiazol Salze
bilden kann, Auftrennung des diastereomeren Salzgemisches in die
Diastereomeren und Freisetzung des gewünschten Enantiomeren bewirkt.

3.      Verfahren nach Anspruch 2, dadurch gekennzeichnet, dass man als
optisch aktive Säure eine gegebenenfalls 0,0'-diacylierte Weinsäure
verwendet.

4.      Verfahren nach Anspruch .2, dadurch gekennzeichnet, dass man als
optisch aktive Säure D-Weinsäure  bzw. zunächst L- und dann D-Weinsäure, 0,0'-Di-p-toluoyl-D-weinsäure oder 0,0'-Di-p-toluoyl-L-Weinsäure
verwendet.

5.      Verfahren nach Anspruch 1, dadurch gekennzeichnet, dass man von
einem (-)-threo-2-(2-X-Aethylthio)-4,5-di-p-methoxyphenyl-imidazolin
ausgeht.

6.      Verfahren nach einem der Ansprüche 1-5, dadurch gekennzeichnet, dass man das linksdrehende Enantiomere des trans-5,6-Di-p-methoxy-phenyl-2,3,5,6-tetrahydro-imidazo[2,1-b]thiazols in freier Form herstellt.

7.      Verfahren nach einem der Ansprüche 1-5, dadurch gekennzeichnet, dass man das                Enantiomere des trans-5,6-Di-p-methoxy-phenyl-2,3,5,6-tetrahydro-imidazo[2,1-b]thiazols in Form der Chlor-wasserstoffäure-, Bromwasserstoffsäure-, Schwefelsäure- oder Phosphor-säuresalzes oder in Form des Salzes mit Essig-, Propion-, Bernstein-, Glykol-, Milch-, Aepfel-, Zitronen-, Ascorbin-, Malein-, Phenylessig-, Benzoe-, 4-Aminobenzoe-, Anthanil-, 4-Hydroxybenzoe-, Salicyl-, Aminosalicyl-, Embon- oder Nicotin-, sowie Methansulfon-, Aethansulfon-, 2-Hydroxyäthansulfon-, Aethylensulfon-, Benzolsulfon-, p-Toluolsulfon-, Naphthalinsulfon-, Sulfanil- oder Cyclohexylsulfaminsäure herstellt.

8.      Verfahren nach einem der Ansprüche 1-5, dadurch gekennzeichnet, dass man das linksdrehende Enantiomere des trans-5,6-Di-p-methoxy-phenyl-2,3,5,6-tetrahydro-imidazo[2,1-b]thiazols in Form eines Salzes mit D- oder L-Weinsäure bzw. eines von der gegebenenfalls durch $C_1$-$C_4$-Alkyl, $C_1$-$C_4$-Alkoxy, Halogen bis und mit Atomnummer 35 und/oder Nitro substituierten Benzoesäure abgeleiteten O,O'-Diacylderivates dersel-ben herstellt.

9.      Verfahren nach einem der Ansprüche 1-5, dadurch gekennzeichnet, dass man das linksdrehende Enantiomere des trans-5,6-Di-p-methoxy-phenyl-2,3,5,6-tetrahydro-imidazo[2,1-b]thiazols in Form des D-Tartrates, des Di-p-Toluoyl-D-tartrates oder des Di-p-Toluol-L-tartrates, herstellt.

10.     Verfahren, dadurch gekennzeichnet, dass man pharmazeutische Präparate enthaltend eine Verbindung gemäss einem der Ansprüche 1 und 6 bis 9 neben üblichen pharmazeutischen Hilfs- und Trägerstoffen her-stellt.

Europäisches
Patentamt

EUROPÄISCHER RECHERCHENBERICHT

0013561

Nummer der Anmeldung

EP 80 10 0035

| Kategorie | Kennzeichnung des Dokuments mit Angabe, soweit erforderlich, der maßgeblichen Teile | betrifft Anspruch |
|---|---|---|
| | DE – A – 2 742 725 (SMITHKLINE CORP.) <br> * Seiten 1-7; Ansprüche * <br><br> -- | 1-7, 10 |
| | GB – A – 1 183 781 (I.C.I.) <br> * Vollständig * <br><br> -- | 1,7-10 |
| P,X | EP – A – 0 000 353 (CIBA-GEIGY) <br> * Seite 9, Absatz 2; Seite 18, Beispiel 7; Seiten 23-26; Ansprüche * <br><br> ---- | 1-10 |

**KLASSIFIKATION DER ANMELDUNG (Int.Cl. )**

C 07 D 513/04
C 07 B 19/00
A 61 K 31/425/
C 07 D 233/42
(C 07 D 513/04
277/00
235/00)

**RECHERCHIERTE SACHGEBIETE (Int. Cl. 3)**

C 07 D 513/04

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde
  liegende Theorien oder
  Grundsätze
E: kollidierende Anmeldung
D: in der Anmeldung angeführtes
  Dokument
L: aus andern Gründen
  angeführtes Dokument
&: Mitglied der gleichen Patentfamilie, übereinstimmendes
  Dokument

Der vorliegende Recherchenbericht wurde für alle Patentansprüche erstellt.

| Recherchenort | Abschlußdatum der Recherche | Prüfer |
|---|---|---|
| Den Haag | 26-03-1980 | ALLARD |